# EUROPEAN PATENT SPECIFICATION

(11) **EP 0 884 319 B1**
(45) Date of publication and mention of the grant of the patent: **06.05.2004**
(21) Application number: 98114130.2
(22) Date of filing: 10.03.1993
(51) Int. Cl.: C07D 498/04, A61K 31/535

(54) **N- (1-nButyl-4-piperidyl)methyl -3,4-dihydro-2H- 1,3 oxazino 3,2-a indole-10-carboxamide or a pharmaceutically acceptable salt thereof**
N- (1-nButyl-4-piperidyl)methyl -3,4-dihydro-2H- 1,3 oxazino 3,2-a indol-10-carboxamid oder ein pharmazeutisch annehmbares Salz davon
N- (1-nButyl-4-piperidyl)methyl -3,4-dihydro-2H- 1,3 oxazino 3,2-a indole-10-carboxamide ou un de ses sels pharmaceutiquement acceptables

(30) Priority: 12.03.1992 GB 9205428; 05.09.1992 GB 9218846; 29.12.1992 GB 9227045
(43) Date of publication of application: 16.12.1998
(62) Divisional of application: 93905561.2
(73) Proprietor: SmithKline Beecham plc, Brentford, Middlesex TW8 9GS (GB)
(72) Inventor: Gaster, Laramie Mary, GlaxoSmithKline, Harlow, Essex CM19 5AW (GB); Wyman, Paul Adrian, GlaxoSmithKline, Harlow, Essex CM19 5AW (GB)
(74) Representative: Giddings, Peter John, Dr.

(56) References cited:
- EP-A- 0 429 984
- EP-A- 0 485 962
- EP-A- 0 501 322
- GB-A- 1 566 307

## Description

This invention relates to a novel compound having pharmacological activity, to a process for its preparation and to its use as a pharmaceutical.

EP-A-429984 (Nisshin Flour Milling Co., Ltd.) describes indole derivatives having 5-HT₃ receptor antagonist activity.

European Journal of Pharmacology 146 (1988), 187-188, and Naunyn-Schmiedeberg's Arch. Pharmacol. (1989) 340:403-410, describe a non classical 5-hydroxytryptamine receptor, now designated the 5-HT₄ receptor, and that ICS 205-930, which is also a 5-HT₃ receptor antagonist, acts as an antagonist at this receptor.

WO 91/16045 (SmithKline and French Laboratories Limited) describes the use of cardiac 5-HT₄ receptor antagonists in the treatment of atrial arrhythmias and stroke.

EP-A-501322 (Glaxo Group Limited) describes indole derivatives having 5-HT₄ antagonist activity.

A novel, structurally distinct compound has now been discovered, which compound is an indole derivative 1,2-disubstituted by alkyleneoxy, with an azacyclic moiety. This compound has 5-HT₄ receptor antagonist activity.

Accordingly, the present invention provides N-[(1-ⁿbutyl-4-piperidyl)methyl]-3,4-dihydro-2H-[1,3]oxazino[3,2-a]indole-10-carboxamide, namely the compound of formula (I), or a pharmaceutically acceptable salt thereof: 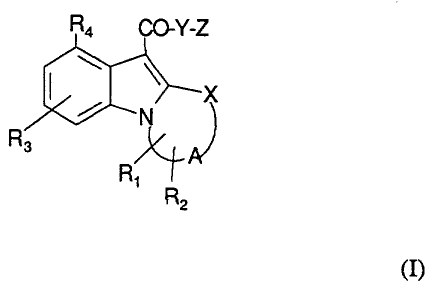 wherein
X is O;
A is -(CH₂)₃- ;
R₁ R₂ R₃ and R₄ are hydrogen;
Y is NH;
Z is of formula (i):

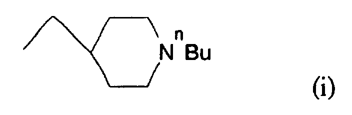

The pharmaceutically acceptable salts of the compound of the formula (I) include acid addition salts with conventional acids such as hydrochloric, hydrobromic, boric, phosphoric, sulphuric acids and pharmaceutically acceptable organic acids such as acetic, tartaric, maleic, citric, succinic, benzoic, ascorbic, methanesulphonic, α-keto glutaric, α-glycerophosphoric, and glucose-1-phosphoric acids.

Examples of pharmaceutically acceptable salts include quaternary derivatives of the compound of formula (I) which are the compound quaternised by compounds Rₓ-T wherein Rₓ is methyl, ethyl, *n*-propyl, *iso*-propyl, benzyl or phenethyl, and T is a radical corresponding to an anion of an acid. Suitable examples of T include halide such as chloride, bromide and iodide.

Examples of pharmaceutically acceptable salts also include internal salts which are N-oxides.

The compound of the formula (I), and its pharmaceutically acceptable salts, (including quaternary derivatives and N-oxides) may also form pharmaceutically acceptable solvates, such as hydrates, which are included wherever the compound of formula (I) or a salt thereof is herein referred to.

The compound of formula (I) may be prepared by conventional coupling of the indole moiety with Z. Suitable methods are as described in GB 2125398A (Sandoz Limited), GB 1593146A and EP-A-36269 (Beecham Group p.l.c.), EP-A-429984 (Nisshin Flour Milling Co.) and EP-A-328200 (Merck Sharp & Dohme Limited). Reference is also made to EP-A-501322 (Glaxo Group Limited). It will be appreciated that the (CH₂)ᵣ-O containing ring or R₃/R₄ introduction/modification may be carried out before or after coupling.

Aza(bi)cyclic side chain intermediates are known compounds or may be prepared according to the methods described in PCT/GB92/01519 and /01612 (SmithKline Beecham p.l.c.).

The compounds of the present invention are 5-HT₄ receptor antagonists and it is thus believed may generally be used in the treatment or prophylaxis of gastrointestinal disorders, cardiovascular disorders and CNS disorders.

They are of potential interest in the treatment of irritable bowel syndrome (IBS), in particular the diarrhoea aspects of IBS, i.e., these compounds block the ability of 5-HT to stimulate gut motility via activation of enteric neurones. In animal models of IBS, this can be conveniently measured as a reduction of the rate of defaecation. They are also of potential use in the treatment of urinary incontinence which is often associated with IBS.

They may also be of potential use in other gastrointestinal disorders, such as those associated with upper gut motility, and as antiemetics. In particular, they are of potential use in the treatment of the nausea and gastric symptoms of gastro-oesophageal reflux disease and dyspepsia. Antiemetic activity is determined in known animal models of cytotoxic-agent/radiation induced emesis.

Specific cardiac 5-HT₄ receptor antagonists which prevent atrial fibrillation and other atrial arrhythmias associated with 5-HT, would also be expected to reduce occurrence of stroke (see A.J. Kaumann 1990, Naumyn-Schmiedeberg's Arch. Pharmacol. 342, 619-622, for appropriate animal test method).

It is believed that platelet-derived 5-HT induces atrial arrhythmias which encourage atrial fibrillation and atrial disorders are associated with symptomatic cerebral and sytemic embolism. Cerebral embolism is the most common cause of ischaemic stroke and the heart the most common source of embolic material. Of particular concern is the frequency of embolism associated with atrial fibrillation.

Anxiolytic activity is likely to be effected via the hippocampus (Dumuis *et al* 1988, Mol Pharmacol., 34, 880-887). Activity may be demonstrated in standard animal models, the social interaction test and the X-maze test.

Migraine sufferers often undergo situations of anxiety and emotional stress that precede the appearance of headache (Sachs, 1985, Migraine, Pan Books, London). It has also been observed that during and within 48 hours of a migraine attack, cyclic AMP levels are considerably increased in the cerebrospinal fluid (Welch *et al*., 1976, Headache 16, 160-167). It is believed that a migraine, including the prodomal phase and the associated increased levels of cyclic AMP are related to stimulation of 5-HT₄ receptors, and hence that administration of a 5-HT₄ antagonist is of potential benefit in relieving a migraine attack.

The invention also provides a pharmaceutical composition comprising the compound of formula (I), or a pharmaceutically acceptable salt thereof, and a pharmaceutically acceptable carrier.

Such compositions are prepared by admixture and are usually adapted for enteral such as oral, nasal or rectal, or parenteral administration, and as such may be in the form of tablets, capsules, oral liquid preparations, powders, granules, lozenges, reconstitutable powders, nasal sprays, suppositories, injectable and infusable solutions or suspensions. Sublingual or transdermal administration is also envisaged. Orally administrable compositions are preferred, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art, for example with an enteric coating.

Suitable fillers for use include cellulose, mannitol, lactose and other similar agents. Suitable disintegrants include starch, polyvinylpolypyrrolidone and starch derivatives such as sodium starch glycollate. Suitable lubricants include, for example, magnesium stearate.

Suitable pharmaceutically acceptable wetting agents include sodium lauryl sulphate. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral liquid preparations are usually in the form of aqueous or oily suspensions, solutions, emulsions, syrups, or elixirs or are presented as a dry product for reconstitution with water or other suitable vehicle before use. Such liquid preparations may contain conventional additives such as suspending agents, emulsifying agents, non-aqueous vehicles (which may include edible oils), preservatives, and flavouring or colouring agents.

The oral compositions may be prepared by conventional methods of blending, filling or tabletting. Repeated blending operations may be used to distribute the active agent throughout those compositions employing large quantities of fillers. Such operations are, of course, conventional in the art.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure of ethylene oxide before suspending in the sterile vehicle. Advantageously, a surfactant or wetting agent is included in the composition to facilitate uniform distribution of the compound of the invention.

The invention further provides the use of the compound of formula (I), or a pharmaceutically acceptable salt thereof, and a pharmaceutically acceptable carrier in the preparation by admixture of a pharmaceutical composition for the treatment of irritable bowel syndrome, gastro-oesophagal reflux disease, dyspepsia, atrial arrhythmias and stroke, anxiety and/or migraine in mammals, such as humans, the composition being for the administration of an effective amount of the compound of the formula (I) or the pharmaceutically acceptable salt thereof. In particular, the composition is for treatment of IBS or atrial arrhythmias and stroke.

An amount effective to treat the disorders hereinbefore described depends on the relative efficacies of the compounds of the invention, the nature and severity of the disorder being treated and the weight of the mammal. However, a unit dose for a 70 kg adult will normally contain 0.05 to 1000 mg for example 0.5 to 500 mg, of the compound of the invention. Unit doses may be administered once or more than once a day, for example, 2, 3 or 4 times a day, more usually 1 to 3 times a day, that is in the range of approximately 0.0001 to 50 mg/kg/day, more usually 0.0002 to 25 mg/kg/day.

No adverse toxicological effects are indicated within the aforementioned dosage ranges.

The invention also provides the compound of formula (I) or a pharmaceutically acceptable salt thereof for use as an active therapeutic substance, in particular for use as a 5-HT₄ receptor antagonist in the treatment of the disorders hereinbefore described.

The invention also provides the use of the compound of formula (I) in the manufacture of a medicament for use as a 5-HT₄ receptor antagonist in the treatment of the disorders hereinbefore described.

The following Example illustrates the preparation of the compound of formula (I); the following Description illustrates the preparation of an intermediate.

### Example

### N-[(1-ⁿButyl-4-piperidyl)methyl]-3,4-dihydro-2H-[1,3]oxazino[3,2-a]indole-10-carboxamide

**Method 1:-** A stirred solution of N-chlorosuccinimide (57 mg, 0.48 mmole) in chloroform (3 ml) was treated with a solution of N-[(1-ⁿbutyl-4-piperidyl)methyl] indole-3-carboxamide (100 mg, 0.32 mmole) in chloroform (8 ml) and kept at room temperature for 2h, then treated with 3-bromo-1-propanol (0.03 ml, 0.32 mmole). After stirring for 16h, more 3-bromo-1-propanol (0.03 ml, 0.32 mmole) was added. The mixture was stirred at room temperature for a further 3h, then treated with excess 10% Na₂CO₃ solution and extracted with chloroform. The extract was dried (Na₂SO₄) and concentrated *in vacuo* to leave a yellow oil, which was dissolved in acetone (10 ml), treated with anhydrous potassium carbonate (130 mg, 0.96 mmole) and stirred at room temperature for 16h. The mixture was concentrated *in vacuo*, the residue treated with 10% Na₂CO₃ solution (10 ml) and extracted with chloroform. The extract was dried (Na₂SO₄) and concentrated *in vacuo* to leave a yellow oil, which was chromatographed, initially on silica gel eluting with chloroform/methanol (19:1), then on basic alumina eluting with ethyl acetate. The colourless oil obtained crystallised from ether to afford the title compound as a white solid (20 mg, 17%) mp 110-113°C.
¹H NMR (CDCl₃)
δ: 8.34 (d,1H), 7.05-7.30 (m,3H), 6.55 (t,1H), 4.53 (t,2H), 4.10 (t,2H), 3.33 (t,2H), 2.90-3.05 (m,2H), 2.25-2.45 (m,4H), 1.90-2.25 (m,2H), 1.20-1.85 (m,9H), 0.92 (t,3H).
MS (CI) MH⁺ 370.

**Method 2:-** A stirred suspension of N-[(1-ⁿbutyl-4-piperidyl)methyl] indole-3-carboxamide (120g, 0.38 mole) in chloroform (2 L) under nitrogen at room temperature was treated with freshly distilled 3-bromo-1-propanol (69 ml, 0.77 mole) followed by the portionwise addition of dry N-chlorosuccinimide (55g, 0.42 mole) over 5 minutes. The resulting yellow solution was stirred for 2.5h, then treated with 1M HCl in ether (15 ml, 0.015 mole). A moderate exotherm occurred and the reaction colour changed to orange. After a further 2h the mixture was treated with 10% Na₂CO₃ solution (700 ml) and the chloroform layer separated, dried (Na₂SO₄) and concentrated *in vacuo* to leave a thick red oil. This was treated with acetone (1.5 L) and anhydrous potassium carbonate (130g, 0.95 mole), then stirred at room temperature for 18h. The reaction mixture was concentrated *in vacuo* and the residue treated with water (1 L) and extracted with ethyl acetate (1 L). On standing a solid began crystallising from the ethyl acetate extract. After 2h at 8°C this was filtered off and dried to afford 51.7g of the title compound (E3) as a beige solid. The mother liquors were extracted with 1M HCl acid (800 ml), the acid extract then basified with K₂CO₃ and extracted with chloroform (2 x 700 ml). The combined chloroform extracts were dried (Na₂SO₄), concentrated *in vacuo* and the residue chromatographed on silica gel eluting with chloroform/methanol (96:4). A yellow oil was obtained which upon trituration with ether gave a further 21.3g of title compound as a white solid. Conversion to the hydrochloride salt and recrystallisation from ethanol/60-80 petrol gave a white solid mp 254-256 °C dec.
HCl salt - ¹H NMR (D₂O)
δ: 7.90 (d,1H), 6.88-7.20 (m,3H), 4.35 (br t,2H), 3.70 (br t,2H), 3.40 (br d,2H), 3.20 (br d,2H), 2.9 (br t,2H), 2.65(br t,2H), 2.12 (br t,2H), 1.20-1.90 (m,9H), 0.87 (t,3H).

Elemental analysis obtained was as follows:

| | Theory | Found | |
|---|---|---|---|
| Carbon | 65.09 | 64.76, | 64.75 |
| Hydrogen | 7.95 | 7.73, | 7.77 |
| Nitrogen | 10.35 | 10.35, | 10.36 |

### Description (intermediate for Example)

### a) N-(1-ⁿButyl-4-piperidyl)methylamine

A stirred solution of isonipecotamide (70g, 0.55 mole) and 1-bromobutane (58.8 ml, 0.55 mole) in ethanol (700 ml) was treated with anhydrous potassium carbonate (152g, 1.10 mole) and heated under reflux for 3h. The mixture was allowed to cool, then filtered and the filtrate concentrated under vacuum. The residual oil was dissolved in chloroform (400 ml) and washed with water (1 x 300 ml), then dried (Na₂SO₄) and concentrated under vacuum to leave a yellow oil (77.5g). This oil was mixed thoroughly with phosphorus pentoxide (75g) and the mixture heated at 160-180°C under nitrogen for 2.5h with gentle stirring. The reaction mixture was allowed to cool, then treated with water (500 ml). When the solid mass had dissolved, the solution was basified by addition of solid K₂CO₃ and extracted with ethyl acetate (2x400 ml). The combined extracts were dried (Na₂SO₄) and concentrated *in vacuo* to leave a brown oil (78g). This was dissolved in dry ether (400 ml) and added dropwise over 30 minutes to a stirred suspension of lithium aluminium hydride (25g, 0.66 mole) in ether (200ml) at 0°C under nitrogen. When addition was complete, the mixture was allowed to warm upto room temperature and stir for 18h. It was recooled to 0°C and treated cautiously with water (25ml), 10% NaOH solution (25 ml) and water again (75ml). The mixture was filtered through kieselguhr and the filtrate concentrated *in vacuo* to leave a brown oil, which was distilled under vacuum to afford the title compound as a colourless oil (66g, 71 %) bp 96-99°C at 3 mm Hg.
¹H NMR (CDCl₃)
δ: 2.90-3.02(m,2H), 2.58(d,2H), 2.25-2.38(m,2H), 1.65-2.00(m,4H), 1.08-1.58(m,9H), 0.92(t,3H).

### b) N-[(1-ⁿButyl-4-piperidyl)methyl] indole-3-carboxamide

To a stirring solution of indole-3-carboxylic acid (1g) in dichloromethane (20 ml) at 0°C under nitrogen was added oxalyl chloride (0.81 ml) and dry dimethylformamide (3 drops). After 3 hours, the solvents were evaporated under reduced pressure. A portion of the residual acid chloride (420 mg) was dissolved in dichloromethane (12 ml) and added dropwise to a solution of N-(1-ⁿbutyl-4-piperidyl)methylamine (400 mg) in dichloromethane (12 ml) followed by triethylamine (0.36 ml). After stirring at ambient temperature overnight, the reaction mixture was washed with saturated NaHCO₃, and the organic phase was dried (Na₂SO₄). The solvent was evaporated under reduced pressure and the residue recrystallised from ethyl acetate to give the title compound (467 mg, 64%).
¹H NMR (CDCl₃) 250 MHz
δ: 9.29 (br s,1H), 8.05-7.9 (m,1H), 7.81 (d,1H), 7.55-7.4 (m,1H), 7.39-7.2 (m,2H), 6.28 (br s,1H), 3.39 (t,2H), 3.0 (br d,2H), 2.45-2.25 (m,2H), 2.1-1.1 (m,11H), 0.9 (t,3H).

### 5-HT₄ RECEPTOR ANTAGONIST ACTIVITY

### 1) Guinea pig colon

Male guinea-pigs, weighing 250-400g are used. Longitudinal muscle-myenteric plexus preparations, approximately 3cm long, are obtained from the distal colon region. These are suspended under a 0.5g load in isolated tissue baths containing Krebs solution bubbled with 5% CO₂ in O₂ and maintained at 37°C. In all experiments, the Krebs solution also contains methiothepin 10⁻⁷M and granisetron 10⁻⁶M to block effects at 5-HT₁, 5-HT₂ and 5-HT₃ receptors.

After construction of a simple concentration-response curve with 5-HT, using 30s contact times and a 15min dosing cycle, a concentration of 5-HT is selected so as to obtain a contraction of the muscle approximately 40-70% maximum(10⁻⁹M approx). The tissue is then alternately dosed every 15min with this concentration of 5-HT and then with an approximately equi-effective concentration of the nicotine receptor stimulant, dimethylphenylpiperazinium (DMPP). After obtaining consistent responses to both 5-HT and DMPP, increasing concentrations of a putative 5-HT₄ receptor antagonist are then added to the bathing solution. The effects of this compound are then determined as a percentage reduction of the contractions evoked by 5-HT or by DMPP. From this data, pIC₅₀ values are determined, being defined as the -log concentration of antagonist which reduces the contraction by 50%. A compound which reduces the response to 5-HT but not to DMPP is believed to act as a 5-HT₄ receptor antagonist.

The compound was generally active in the range of concentrations of the order of pIC₅₀=7 or more.

### 2) Piglet Atria

The compound was tested in the piglet spontaneous beating screen (Naunyn-Schmiedeberg's Arch. Pharmacol 342, 619-622). pK_{B} (-log₁₀ K_{B}) value for the compound of the Example was 10.05.

### 3) Rat oesophagus

Rat oesophageal tunica muscularis mucosae is set up according to Baxter *et. al*. Naunyn-Schmiedeberg's Arch. Pharmacol., 343, 439-446 (1991). The inner smooth muscle tube of the muscularis mucosae is isolated and mounted for isometric tension recording in oxygenated (95% O₂/5% CO₂) Tyrodes solution at 37°C. All experiments are performed in pargyline pre-treated preparations (100µM for 15 min followed by washout) and in the presence of cocaine (30µM). Relaxant responses to 5-HT are obtained after pre-contracting the oesophagus tissue with carbachol (3µM).

### 4) 5-HT-induced motility in dog gastric pouch

The compound is tested for inhibition in the *in vivo* method described in "Stimulation of canine motility by BRL 24924, a new gastric prokinetic agent", Bermudez *et al*, J. Gastrointestinal Motility, 1990, 2(4), 281-286.

### IN VIVO TESTING FOR ANXIOLYTIC ACTIVITY

### Social Interaction Test in Rats

Rats (male, Sprague Dawleys, Charles River, 250-300g) are housed in groups of eight in a holding room for 5 days. They are then housed singly in a room adjacent to the experimental room for 4 days prior to the experimental day. On the experimental day rats are administered vehicle, test compound or a benzodiazepine anxiolytic, chlordiazepoxide, p.o. in pairs (n=8-16), at 15 minute intervals beginning at 10.00 a.m. 30 mins. later they are placed with a weight matched pair-mate (encountered for the first time) in the social interaction box in a separate room. The box is made of white perspex 54 cm x 37 cm x 26 cm with a transparent perspex front side and no lid. The floor is divided up into 24 squares and the box is brightly lit (115 lux). Active social interactive behaviours (grooming, sniffing, climbing over or under, following, biting, mounting and boxing) are scored blind for the next 15 min by remote video monitoring to give total interaction scores. The number of squares crossed by each rat is also scored and summed. After the end of each test the box is carefully wiped.

The compound of the Example increased total interaction scores over the dose range 0.01 10 mg/kg p.o.

## Claims

1. N-[(1-ⁿButyl-4-piperidyl)methyl]-3,4-dihydro-2H-[1,3]oxazino[3,2-a]indole-10-carboxamide or a pharmaceutically acceptable salt thereof.

2. N-[(1-ⁿButyl-4-piperidyl)methyl]-3,4-dihydro-2H-[1,3]oxazino[3,2-a]indole-10-carboxamide.

3. A pharmaceutically acceptable salt according to claim 1, which is an acid addition salt with hydrochloric, hydrobromic, boric, phosphoric, or sulphuric acid, or with a pharmaceutically acceptable organic acid.

4. A pharmaceutically acceptable salt according to claim 1, which is an acid addition salt with hydrochloric, hydrobromic, boric, phosphoric, sulphuric, acetic, tartaric, maleic, citric, succinic, benzoic, ascorbic, methanesulphonic, α-keto glutaric, α-glycerophosphoric, or glucose-1-phosphoric acid.

5. The hydrochloride salt of N-[(1-ⁿButyl-4-piperidyl)methyl]-3,4-dihydro-2H-[1,3]oxazino[3,2-a]indole-10-carboxamide.

6. A pharmaceutical composition comprising N-[(1-ⁿbutyl-4-piperidyl)methyl]-3,4-dihydro-2H-[1,3]oxazino[3,2-a]indole-10-carboxamide or a pharmaceutically acceptable salt thereof, according to any of claims 1 to 5, and a pharmaceutically acceptable carrier.

7. A composition according to claim 6 being a unit dose for a 70 kg adult containing 0.05 to 1000mg of a compound according to any of claims 1 to 5.

8. A composition according to claim 7 being a unit dose for a 70 kg adult containing 0.5 to 500mg of a compound according to any of claims 1 to 5,

9. A composition according to any of claims 6, 7 or 8, prepared by admixture and adapted for oral administration.

10. A composition according to claim 9 in the form of a tablet or capsule.

11. N-[(1-ⁿbutyl-4-piperidyl)methyl]-3,4-dihydro-2H-[1,3]oxazino[3,2-a]indole-10-carboxamide or a pharmaceutically acceptable salt thereof, according to any of claims 1 to 5, for use as an active therapeutic substance.

12. N-[(1-ⁿbutyl-4-piperidyl)methyl]-3,4-dihydro-2H-[1,3]oxazino[3,2-a]indole-10-carboxamide or a pharmaceutically acceptable salt thereof, according to claim 11, for use as a 5-HT₄ receptor antagonist in the treatment of irritable bowel syndrome, gastro-oesophagal reflux disease, dyspepsia, atrial arrhythmias and stroke, anxiety and/or migraine in mammals.

13. N-[(1-ⁿbutyl-4-piperidyl)methyl]-3,4-dihydro-2H-[1,3]oxazino[3,2-a]indole-10-carboxamide or a pharmaceutically acceptable salt thereof, according to claim 11, for use as a 5-HT₄ receptor antagonist in the treatment of atrial arrhythmias and stroke in mammals.

14. N-[(1-ⁿbutyl-4-piperidyl)methyl]-3,4-dihydro-2H-[1,3]oxazino[3,2-a]indole-10-carboxamide or a pharmaceutically acceptable salt thereof, according to claim 11, for use as a 5-HT₄ receptor antagonist in the treatment of atrial arrhythmias and stroke in humans.

15. N-[(1-ⁿbutyl-4-piperidyl)methyl]-3,4-dihydro-2H-[1,3]oxazino[3,2-a]indole-10-carboxamide or a pharmaceutically acceptable salt thereof, according to claim 11, for use as a 5-HT₄ receptor antagonist in the treatment of urinary incontinence.

16. The use of N-[(1-ⁿbutyl-4-piperidyl)methyl]-3,4-dihydro-2H-[1,3]oxazino[3,2-a]indole-10-carboxamide in the manufacture of a medicament for use as a 5-HT₄ receptor antagonist in the treatment or prophylaxis of gastrointestinal disorders, cardiovascular disorders and CNS disorders.

17. The use of N-[(1-ⁿbutyl-4-piperidyl)methyl]-3,4-dihydro-2H-[1,3]oxazino[3,2-a]indole-10-carboxamide in the manufacture of a medicament for use as a 5-HT₄ receptor antagonist in the treatment of irritable bowel syndrome, gastro-oesophagal reflux disease, dyspepsia, atrial arrhythmias and stroke, anxiety and/or migraine in mammals.

18. The use of N-[(1-ⁿbutyl-4-piperidyl)methyl]-3,4-dihydro-2H-[1,3]oxazino[3,2-a]indole-10-carboxamide or a pharmaceutically acceptable salt thereof, as defined in any of claims 1 to 5, and a pharmaceutically acceptable carrier in the preparation by admixture of a pharmaceutical composition as defined in claim 6,
wherein the pharmaceutical composition is for the treatment of irritable bowel syndrome, gastro-oesophagal reflux disease, dyspepsia, atrial arrhythmias and stroke, anxiety and/or migraine in mammals, and wherein the pharmaceutical composition is for administration of an effective amount of the N-[(1-ⁿbutyl-4-piperidyl)methyl]-3,4-dihydro-2H-[1,3]oxazino[3,2-a]indole-10-carboxamide or the pharmaceutically acceptable salt thereof.

19. The use according to claim 18, wherein the pharmaceutical composition is a unit dose for a 70 kg adult containing 0.5 to 500mg of a compound according to any of claims 1 to 5.

20. The use according to claim 18 or 19, wherein the pharmaceutical composition is a unit dose for administration 1 to 3 times a day in the range of 0.0002 to 25 mg/kg/day.

21. The use according to claim 18, 19 or 20, wherein the pharmaceutical composition is adapted for oral administration.

22. The use according to claim 21, wherein the pharmaceutical composition is in the form of a tablet or capsule.

23. The use according to claim 17, 18, 19, 20, 21 or 22, wherein the medicament or the pharmaceutical composition is for the treatment of atrial arrhythmias and stroke in mammals.

24. The use according to claim 17, 18, 19, 20, 21 or 22, wherein the medicament or the pharmaceutical composition is for the treatment of atrial arrhythmias and stroke in humans.

25. The use according to claim 17, 18, 19, 20, 21 or 22, wherein the medicament or the pharmaceutical composition is for the treatment of irritable bowel syndrome in humans.

26. The use of N-[(1-ⁿbutyl-4-piperidyl)methyl]-3,4-dihydro-2H-[1,3]oxazino[3,2-a]indole-10-carboxamide in the manufacture of a medicament for use as a 5-HT₄ receptor antagonist in the treatment of urinary incontinence.

## Patentansprüche

1. N-[(1-ⁿButyl-4-piperidyl)methyl]-3,4-dihydro-2H-[1,3]oxazino[3,2-a]indol-10-carboxamid oder ein pharmazeutisch verträgliches Salz davon.

2. N-[(1-ⁿButyl-4-piperidyl)methyl]-3,4-dihydro-2H-[1,3]oxazino[3,2-a]indol-10-carboxamid.

3. Pharmazeutisch verträgliches Salz gemäß Anspruch 1, welches ein Säureadditionssalz mit Chlorwasserstoffsäure, Bromwasserstoffsäure, Borsäure, Phosphorsäure oder Schwefelsäure oder mit einer pharmazeutisch verträglichen organischen Säure ist.

4. Pharmazeutisch verträgliches Salz gemäß Anspruch 1, welches ein Säureadditionssalz mit Chlorwasserstoffsäure, Bromwasserstoffsäure, Borsäure, Phosphorsäure, Schwefelsäure, Essigsäure, Weinsäure, Maleinsäure, Zitronensäure, Bernsteinsäure, Benzoesäure, Ascorbinsäure, Methansulfonsäure, α-Ketoglutarsäure, α-Glycerophosphorsäure oder Glucose-1-phosphorsäure ist.

5. Hydrochloridsalz von N-[(1-ⁿButyl-4-piperidyl)methyl]-3,4-dihydro-2H-[1,3]oxazino[3,2-a]indol-10-carboxamid.

6. Arzneimittel, umfassend N-[(1-ⁿButyl-4-piperidyl)methyl]-3,4-dihydro-2H-[1,3]-oxazino[3,2-a]indol-10-carboxamid oder ein pharmazeutisch verträgliches Salz davon gemäß einem der Ansprüche 1 bis 5, und einen pharmazeutisch verträglichen Träger.

7. Arzneimittel gemäß Anspruch 6, welches eine Einheitsdosis für einen 70 kg schweren Erwachsenen ist, welches 0,05 bis 1000 mg einer Verbindung gemäß einem der Ansprüche 1 bis 5 enthält.

8. Arzneimittel gemäß Anspruch 7, welches eine Einheitsdosis für einen 70 kg schweren Erwachsenen ist, welches 0,5 bis 500 mg einer Verbindung gemäß einem der Ansprüche 1 bis 5 enthält.

9. Arzneimittel gemäß einem der Ansprüche 6, 7 oder 8, welches durch Beimischen hergestellt und für die orale Verabreichung geeignet ist.

10. Arzneimittel gemäß Anspruch 9 in Tabletten- oder Kapselform.

11. N-[(1-ⁿButyl-4-piperidyl)methyl]-3,4-dihydro-2H-[1,3]oxazino[3,2-a]indol-10-carboxamid oder ein pharmazeutisch verträgliches Salz davon gemäß einem der Ansprüche 1 bis 5 zur Verwendung als eine therapeutisch wirksame Substanz.

12. N-[(1-ⁿButyl-4-piperidyl)methyl]-3,4-dihydro-2H-[1,3]oxazino[3,2-a]indol-10-carboxamid oder ein pharmazeutisch verträgliches Salz davon gemäß Anspruch 11 zur Verwendung als ein 5-HT₄-Rezeptorantagonist bei der Behandlung von Reizkolon, Gastro-Ösophagus-Refluxerkrankung, Dyspepsie, Vorhofarrhythmie und Schlaganfall, Angst und/oder Migräne bei Säugern.

13. N-[(1-ⁿButyl-4-piperidyl)methyl]-3,4-dihydro-2H-[1,3]oxazino[3,2-a]indol-10-carboxamid oder ein pharmazeutisch verträgliches Salz davon gemäß Anspruch 11 zur Verwendung als ein 5-HT₄-Rezeptorantagonist bei der Behandlung von Vorhofarrhythmie und Schlaganfall bei Säugern.

14. N-[(1-ⁿButyl-4-piperidyl)methyl]-3,4-dihydro-2H-[1,3]oxazino[3,2-a]indol-10-carboxamid oder ein pharmazeutisch verträgliches Salz davon gemäß Anspruch 11 zur Verwendung als ein 5-HT₄-Rezeptorantagonist bei der Behandlung von Vorhofarrhythmie und Schlaganfall bei Menschen.

15. N-[(1-ⁿButyl-4-piperidyl)methyl]-3,4-dihydro-2H-[1,3]oxazino[3,2-a]indol-10-carboxamid oder ein pharmazeutisch verträgliches Salz davon gemäß Anspruch 11 zur Verwendung als ein 5-HT₄-Rezeptorantagonist bei der Behandlung von Harninkontinenz.

16. Verwendung von N-[(1-ⁿButyl-4-piperidyl)methyl]-3,4-dihydro-2H-[1,3]oxazino[3,2-a]indol-10-carboxamid zur Herstellung eines Medikaments zur Verwendung als ein 5-HT₄-Rezeptorantagonist bei der Behandlung oder Vorbeugung von Gastrointestinal-Störungen, Herzkreislauf-Störungen und ZNS-Störungen.

17. Verwendung von N-[(1-ⁿButyl-4-piperidyl)methyl]-3,4-dihydro-2H-[1,3]oxazino[3,2-a]indol-10-carboxamid zur Herstellung eines Medikaments zur Verwendung als ein 5-HT₄-Rezeptorantagonist bei der Behandlung von Reizkolon, Gastro-Ösophagus-Refluxerkrankung, Dyspepsie, Vorhofarrhythmie und Schlaganfall, Angst und/oder Migräne bei Säugern.

18. Verwendung von N-[(1-ⁿButyl-4-piperidyl)methyl]-3,4-dihydro-2H-[1,3]oxazino[3,2-a]indol-10-carboxamid oder eines pharmazeutisch verträglichen Salzes davon, wie in einem der Ansprüche 1 bis 5 definiert, und eines pharmazeutisch verträglichen Trägers bei der Herstellung durch Beimischen eines Arzneimittels wie in Anspruch 6 definiert, wobei das Arzneimittel zur Behandlung von Reizkolon, Gastro-Ösophagus-Refluxerkrankung, Dyspepsie, Vorhofarrhythmie und Schlaganfall, Angst und/oder Migräne bei Säugern bestimmt ist und wobei das Arzneimittel zur Verabreichung einer wirksamen Menge des N-[(1-ⁿButyl-4-piperidyl)methyl]-3,4-dihydro-2H-[1,3]-oxazino[3,2-a]indol-10-carboxamids oder des pharmazeutisch verträglichen Salzes davon bestimmt ist.

19. Verwendung gemäß Anspruch 18, wobei das Arzneimittel eine Einheitsdosis für einen 70 kg schweren Erwachsenen ist, welche 0,5 bis 500 mg einer Verbindung gemäß einem der Ansprüche 1 bis 5 enthält.

20. Verwendung gemäß Anspruch 18 oder 19, wobei das Arzneimittel eine Einheitsdosis zur Verabreichung 1 bis 3 Mal täglich im Bereich von 0,0002 bis 25 mg/kg/Tag ist.

21. Verwendung gemäß Anspruch 18, 19 oder 20, wobei das Arzneimittel für die orale Verabreichung geeignet ist.

22. Verwendung gemäß Anspruch 21, wobei das Arzneimittel in Tabletten- oder Kapselform vorliegt.

23. Verwendung gemäß Anspruch 17, 18, 19, 20, 21 oder 22, wobei das Medikament oder das Arzneimittel zur Behandlung von Vorhofarrhythmie und Schlaganfall bei Säugern bestimmt ist.

24. Verwendung gemäß Anspruch 17, 18, 19, 20, 21 oder 22, wobei das Medikament oder das Arzneimittel zur Behandlung von Vorhofarrhythmie und Schlaganfall bei Menschen bestimmt ist.

25. Verwendung gemäß Anspruch 17, 18, 19, 20, 21 oder 22, wobei das Medikament oder das Arzneimittel zur Behandlung von Reizkolon bei Menschen bestimmt ist.

26. Verwendung von N-[(1-ⁿButyl-4-piperidyl)methyl]-3,4-dihydro-2H-[1,3]oxazino [3,2-a]indol-10-carboxamid zur Herstellung eines Medikaments zur Verwendung als ein 5-HT₄-Rezeptorantagonist bei der Behandlung von Harninkontinenz.

## Revendications

1. N-[(1-ⁿbutyl-4-pipéridyl)méthyl]-3,4-dihydro-2H-[1,3]oxazino[3,2-a]indole-10-carboxamide ou un de ses sels pharmaceutiquement acceptables.

2. N-[(1-ⁿbutyl-4-pipéridyl)méthyl]-3,4-dihydro-2H-[1,3]oxazino[3,2-a]indole-10-carboxamide.

3. Sel pharmaceutiquement acceptable suivant la revendication 1, qui est un sel d'addition d'acide formé avec l'acide chlorhydrique, bromhydrique, borique, phosphorique ou sulfurique, ou avec un acide organique pharmaceutiquement acceptable.

4. Sel pharmaceutiquement acceptable suivant la revendication 1, qui est un sel d'addition d'acide formé avec l'acide chlorhydrique, bromhydrique, borique, phosphorique, sulfurique, acétique, tartrique, maléique, citrique, succinique, benzoïque, ascorbique, méthanesulfonique, α-céto-glutarique, α-glycérophosphorique ou glucose-1-phosphorique.

5. Sel chlorhydrate de N-[(1-ⁿbutyl-4-pipéridyl)méthyl]-3,4-dihydro-2H-[1,3]oxazino[3,2-a]indole-10-carboxamide.

6. Composition pharmaceutique comprenant du N-[(1-ⁿbutyl-4-pipéridyl)méthyl]-3,4-dihydro-2H-[1,3]oxazino[3,2-a]indole-10-carboxamide ou un de ses sels pharmaceutiquement acceptables, suivant l'une quelconque des revendications 1 à 5, et un support pharmaceutiquement acceptable.

7. Composition suivant la revendication 6, consistant en une dose unitaire, pour un adulte de 70 kg, contenant 0,05 à 1000 mg d'un composé suivant l'une quelconque des revendications 1 à 5.

8. Composition suivant la revendication 7, consistant en une dose unitaire, pour un adulte de 70 kg, contenant 0,5 à 500 mg d'un composé suivant l'une quelconque des revendications 1 à 5.

9. Composition suivant l'une quelconque des revendications 6, 7 et 8, préparée par mélange et adaptée à l'administration orale.

10. Composition suivant la revendication 9, sous forme d'un comprimé ou d'une capsule.

11. N-[(1-ⁿbutyl-4-pipéridyl)méthyl]-3,4-dihydro-2H-[1,3]oxazino[3,2-a]indole-10-carboxamide ou un de ses sels pharmaceutiquement acceptables, suivant l'une quelconque des revendications 1 à 5, destiné à être utilisé comme substance thérapeutique active.

12. N-[(1-ⁿbutyl-4-pipéridyl)méthyl]-3,4-dihydro-2H-[1,3]oxazino[3,2-a]indole-10-carboxamide ou un de ses sels pharmaceutiquement acceptables, suivant la revendication 11, destiné à être utilisé comme antagoniste des récepteurs 5-HT₄ dans le traitement du syndrome du côlon irritable, de la maladie de reflux gastro-oesophagien, de la dyspepsie, des arythmies auriculaires et d'un ictus, de l'anxiété et/ou de la migraine chez des mammifères.

13. N-[(1-ⁿbutyl-4-pipéridyl)méthyl]-3,4-dihydro-2H-[1,3]oxazino[3,2-a]indole-10-carboxamide ou un de ses sels pharmaceutiquement acceptables, suivant la revendication 11, destiné à être utilisé comme antagoniste des récepteurs 5-HT₄ dans le traitement des arythmies auriculaires et d'un ictus chez des mammifères.

14. N-[(1-ⁿbutyl-4-pipéridyl)méthyl]-3,4-dihydro-2H-[1,3]oxazino[3,2-a]indole-10-carboxamide ou un de ses sels pharmaceutiquement acceptables, suivant la revendication 11, destiné à être utilisé comme antagoniste des récepteurs 5-HT₄ dans le traitement des arythmies auriculaires et d'un ictus chez l'homme.

15. N-[(1-ⁿbutyl-4-pipéridyl)méthyl]-3,4-dihydro-2H-[1,3]oxazino[3,2-a]indole-10-carboxamide ou un de ses sels pharmaceutiquement acceptables, suivant la revendication 11, destiné à être utilisé comme antagoniste des récepteurs 5-HT₄ dans le traitement de l'incontinence urinaire.

16. Utilisation du N-[(1-ⁿbutyl-4-pipéridyl)méthyl]-3,4-dihydro-2H-[1,3]oxazino[3,2-a]indole-10-carboxamide dans la production d'un médicament destiné à être utilisé comme antagoniste des récepteurs 5-HT₄ dans le traitement ou la prophylaxie de troubles gastro-intestinaux, de troubles cardiovasculaires et de troubles du SNC.

17. Utilisation du N-[(1-ⁿbutyl-4-pipéridyl)méthyl]-3,4-dihydro-2H-[1,3]oxazino[3,2-a]indole-10-carboxamide dans la production d'un médicament destiné à être utilisé comme antagoniste des récepteurs 5-HT₄ dans le traitement du syndrome du côlon irritable, de la maladie des reflux gastro-oesophagiens, de la dyspepsie, des arythmies auriculaires et d'un ictus, de l'anxiété et/ou de la migraine chez des mammifères.

18. Utilisation du N-[(1-ⁿbutyl-4-pipéridyl)méthyl]-3,4-dihydro-2H-[1,3]oxazino[3,2-a]indole-10-carboxamide ou d'un de ses sels pharmaceutiquement acceptables, suivant l'une quelconque des revendications 1 à 5, et d'un support pharmaceutiquement acceptable dans la préparation par mélange d'une composition pharmaceutique suivant la revendication 6,
dans laquelle la composition pharmaceutique est destinée au traitement du syndrome du côlon irritable, de la maladie du reflux gastro-oesophagien, de la dyspepsie, des arythmies auriculaires et d'un ictus, de l'anxiété et/ou de la migraine chez des mammifères, et dans laquelle la composition pharmaceutique est destinée à l'administration d'une quantité efficace du N-[(1-ⁿbutyl-4-pipéridyl)méthyl]-3,4-dihydro-2H-[1,3]oxazino[3,2-a]indole-10-carboxamide ou de son sel pharmaceutiquement acceptable.

19. Utilisation suivant la revendication 18, dans laquelle la composition pharmaceutique est une dose unitaire, pour un adulte de 70 kg, contenant 0, 5 à 500 mg d'un composé suivant l'une quelconque des revendications 1 à 5.

20. Utilisation suivant la revendication 18 ou 19, dans laquelle la composition pharmaceutique est une dose unitaire destinée à l'administration 1 à 3 fois par jour dans l'intervalle de 0,0002 à 25 mg/kg/jour.

21. Utilisation suivant la revendication 18, 19 ou 20, dans laquelle la composition pharmaceutique est adaptée à l'administration orale.

22. Utilisation suivant la revendication 21, dans laquelle la composition pharmaceutique est sous forme d'un comprimé ou d'une capsule.

23. Utilisation suivant la revendication 17, 18, 19, 20, 21 ou 22, dans laquelle le médicament ou la composition pharmaceutique est destiné au traitement des arythmies auriculaires et d'un ictus chez des mammifères.

24. Utilisation suivant la revendication 17, 18, 19, 20, 21 ou 22, dans laquelle le médicament ou la composition pharmaceutique est destiné au traitement des arythmies auriculaires et d'un ictus chez l'homme.

25. Utilisation suivant la revendication 17, 18, 19, 20, 21 ou 22, dans laquelle le médicament ou la composition pharmaceutique est destiné au traitement du syndrome du côlon irritable chez l'homme.

26. Utilisation du N-[(1-ⁿbutyl-4-pipéridyl)méthyl]-3,4-dihydro-2H-[1,3]oxazino[3,2-a]indole-10-carboxamide dans la production d'un médicament destiné à être utilisé comme antagoniste des récepteurs 5-HT₄ dans le traitement de l'incontinence urinaire.
